# EUROPEAN PATENT APPLICATION

(11) **EP 4 779 023 A1**
(43) Date of publication of application: **22.07.2026**
(21) Application number: 24865443.6
(22) Date of filing: 10.09.2024
(51) Int. Cl.: C12N 15/864, C12N 5/10, C12N 7/01, C12N 15/35

(54) **AAV VARIANT CAPABLE OF INFECTING INNER EAR**

(30) Priority: 11.09.2023 JP 2023147168
(71) Applicant: Juntendo Educational Foundation, Tokyo 113-8421 (JP); Takara Bio Inc., Kusatsu-shi Shiga 525-0058 (JP)
(72) Inventor: KAMIYA, Kazusaku, Tokyo 113-8421 (JP); TANAKA, Yoshinori, Kusatsu-shi, Shiga 525-0058 (JP); ENOKI, Tatsuji, Kusatsu-shi, Shiga 525-0058 (JP); MIURA, Toshiyuki, Kusatsu-shi, Shiga 525-0058 (JP); NARIKI, Hiroaki, Kusatsu-shi, Shiga 525-0058 (JP); OKAMOTO, Sachiko, Kusatsu-shi, Shiga 525-0058 (JP)
(74) Representative: Schnappauf, Georg
(86) International application number: PCT/JP2024/032382
(87) International publication number: WO 2025/057943

(57) **Abstract**

The present invention provides: a nucleic acid encoding an adeno-associated virus (AAV) capsid protein variant containing a peptide comprising an amino acid sequence selected from the group consisting of SEQ ID NO: 1 to SEQ ID NO: 7 or a peptide comprising an amino acid sequence having a structure that one or several amino acid residues are substituted, deleted, inserted and/or added in an amino acid sequence selected from the group consisting of SEQ ID NO: 1 to SEQ ID NO: 7; and others.

## Description

### TECHNICAL FIELD

The present invention relates to a nucleic acid encoding a mutant of an adeno-associated virus (AAV) capsid protein, the capsid protein mutant having infectivity for the inner ear, an AAV particle comprising the capsid protein mutant, and a method of producing a gene-transduced cell by use of the particle.

### BACKGROUND ART

AAV is a virus having a linear single-stranded DNA genome of 4.7 kb, comprising open reading frames of two genes rep and cap. The rep gene encodes four proteins necessary for genome replication (Rep78, Rep68, Rep52, and Rep40). The cap gene encodes three capsid proteins that assemble for formation of a viral capsid (VP1, VP2, VP3), and assembly-activating protein (AAP). Replication of AAV in nature relies on the presence of a helper virus such as an adenovirus or a herpes virus. In the absence of a helper virus, the genome of AAV is maintained in an episome or integrated into a chromosome of a host, so that the AAV is present in a latent state. Over one hundred serotypes and clades (non-patent document 1) of AAV are currently identified. Particularly, development of vectors for gene delivery based on AAV2 is advanced.

In 1989, a gene delivery vector system based on AAV2 was developed for the first time. Vectors based on AAV have been found to have many advantages. Since wild-type AAV is nonpathogenic and has no etiological relation to any known diseases, vectors based on AAV are believed to be extremely safe. In addition, AAV has high gene transduction efficiency.

Administration of AAV particles enables long-period and stable gene transduction into various target organs and target cells. Until now, gene transduction with high efficiency into skeletal muscles, liver (hepatic cells), heart (cardiac muscle cells), nerve cells, pancreatic gland cells, and pancreatic islet cells has been reported. In addition, AAV has been used in human clinical trials. On the other hand, an attempt to change the cell tropism of AAV by alteration of capsid proteins of the AAV and an attempt to avoid removal of AAV particles by neutralizing antibodies have been made. For example, AAV capsids with tropism for specific organs and cells such as neuroglia cells, airway epithelial cells, coronary artery vascular endothelial cells, and lung, and AAV capsids with tropism for tumor cells such as glioblastoma, melanoma, lung cancer, and breast cancer have been created (non-patent document 2).

Various genetic abnormalities are known in sensorineural hearing loss caused by abnormalities in inner ear cells, such as hereditary hearing loss. However, there is no definitive treatment of sensorineural hearing loss. It is expected that biopharmaceuticals will enable definitive treatment of many causes of sensorineural hearing loss. Expectations are particularly high for AAV vectors having high gene transfer efficiency and low pathogenicity. AAV has been studied as a vector for inner ear gene therapy, and it has been suggested that different viral serotypes of AAV have different tropisms for various cells in the inner ear tissue (hair cells, supporting cells, neurons, fibrocytes, etc.).

WO2006/033689 (patent document 1) discloses a method for transducing mammalian cochlear hair cells or supporting cells using an AAV containing exogenous DNA and a promoter operably linked to the DNA.

WO2017/100791 (patent document 2) discloses an AAV vector containing an Anc80 capsid protein and a transgene, which is useful for efficiently delivering nucleic acids to cochlear and vestibular cells.

WO2019/173367 (patent document 3) discloses that an AAV9-PHP.B vector, in which the amino acid sequence TLAVPFK is inserted at position 588 of the AAV9 capsid protein, efficiently transduces inner and outer hair cells.

WO2020/132578 (patent document 4) discloses a recombinant AAV virion containing an AAV capsid protein in which a peptide containing the amino acid sequence LGETTRP is inserted between amino acids 587 and 588 of AAV2-VP1, and a heterologous nucleic acid for alleviating hearing loss or dizziness.

WO2021/150850 (patent document 5) discloses a recombinant AAV virion comprising an AAV capsid protein in which a mutation in the corresponding parent AAV capsid protein is between amino acids corresponding to amino acids 587 to 595 of AAV8-VP1, and a heterologous nucleic acid for alleviating hearing loss or dizziness.

### PRIOR ART DOCUMENT

### PATENT DOCUMENT

Patent Document 1: WO2006/033689
Patent Document 2: WO2017/100791
Patent Document 3: WO2019/173367
Patent Document 4: WO2020/132578
Patent Document 5: WO2021/150850

### NON-PATENT DOCUMENT

Non-patent Document 1: Gao et al., J. Virology, Vol. 78, pp. 6381-6388, 2004
Non-patent Document 2: Adachi K. et al., Gene Ther. Regul., Vol. 5, pp. 31-55, 2010

### SUMMARY OF THE INVENTION

### PROBLEMS TO BE SOLVED BY THE INVENTION

Objections of the present invention includes provision of an AAV capsid protein mutant with infectivity for the inner ear, and provision of a method of efficiently introducing a gene into the inner ear.

### SOLUTIONS TO THE PROBLEMS

The present inventors intensively made efforts to solve the above-described problems, and as a result, created an AAV capsid protein mutant comprising a peptide containing an amino acid sequence selected from the group consisting of SEQ ID NOs: 1-7. Thus, the present invention was completed.

The present invention generally relates to:
[1] A nucleic acid encoding a mutant of an adeno-associated virus (AAV) capsid protein, the mutant comprising a peptide comprising an amino acid sequence selected from the group consisting of SEQ ID NOs: 1-7 or a peptide comprising an amino acid sequence that differs from an amino acid sequence selected from the group consisting of SEQ ID NOs: 1-7 by substitution, deletion, insertion and/or addition of one or several amino acids;
[2] The nucleic acid according to [1], wherein the AAV capsid protein is derived from AAV2;
[3] The nucleic acid according to [2], wherein the peptide is placed at a position between amino acid number 588 and amino acid number 589 in VP1 of AAV2;
[4] A recombinant DNA comprising the nucleic acid according to any one of [1] to [3];
[5] A cell comprising the nucleic acid according to any one of [1] to [3];
[6] An AAV particle comprising a mutant of an AAV capsid protein which comprises a peptide comprising an amino acid sequence selected from the group consisting of SEQ ID NOs: 1-7 or a peptide comprising an amino acid sequence that differs from an amino acid sequence selected from the group consisting of SEQ ID NOs: 1-7 by substitution, deletion, insertion and/or addition of one or several amino acids;
[7] The AAV particle according to [6], wherein the AAV capsid protein is derived from AAV2;
[8] The AAV particle according to [7], wherein the peptide is placed at a position between amino acid number 588 and amino acid number 589 in VP1 of AAV2;
[9] A method of producing a gene-transduced cell, the method comprising a step of bringing an AAV particle comprising a mutant of an AAV capsid protein which comprises a peptide comprising an amino acid sequence selected from the group consisting of SEQ ID NOs: 1-7 or a peptide comprising an amino acid sequence that differs from an amino acid sequence selected from the group consisting of SEQ ID NOs: 1-7 by substitution, deletion, insertion and/or addition of one or several amino acids, into contact with a cell;
[10] The method according to [9], wherein the AAV capsid protein is derived from AAV2;
[11] The method according to [10], wherein the peptide is placed at a position between amino acid number 588 and amino acid number 589 in VP1 of AAV2;
[12] A method of producing an AAV particle, the method comprising using the cell according to [5] as a host;
[13] A pharmaceutical composition comprising the AAV particle according to any one of [6] to [8], and a heterologous polynucleotide harbored inside the AAV particle.

### EFFECTS OF THE INVENTION

According to the present invention, a gene transduction system useful for gene transduction into the inner ear is provided. The AAV particle of the present invention has high cell tropism for the inner ear, and thus a gene transduced by the AAV particle can be strongly expressed in the inner ear.

### BRIEF DESCRIPTION OF THE DRAWINGS

[FIG. 1] FIG. 1 shows a method for producing a nucleic acid construct that contains a random peptide in a capsid protein.
[FIG. 2] FIG. 2 shows the results of infection into cultured mouse cochlear tissue in Example 5.
[FIG. 3] FIG. 3 shows black and white images converted by using image analysis software (ImageJ).
[FIG. 4] FIG. 4 shows the infection areas of AAV vectors.
[FIG. 5] FIG. 5 shows the infection ratios of SC and HC.

### MODE FOR CARRYING OUT THE INVENTION

As used herein, the "adeno-associated virus" refers to a small virus belonging to the genus *Dependovirus* which lies within the family *Parvoviridae* and capable of infecting primates including human and other mammals. Hereinafter, the adeno-associated virus is abbreviated as AAV. AAV has a non-enveloped shell (capsid) of a regular icosahedron and a linear single-stranded DNA inside the shell. As used herein, AAV includes the wild-type virus and derivatives thereof, and includes all serotypes and clades of AAV unless specified otherwise.

The "vector" as used herein means a molecule or an associated molecule that is used for mediating delivery of a polynucleotide to a cell and which comprises the polynucleotide or associates with the polynucleotide. Examples of the vector include vector DNAs such as plasmid vectors and phage vectors, viral vector particles, liposomes, and other vehicles for gene delivery, unless specified otherwise.

The "capsid protein" as used herein means a protein that is encoded by the cap gene present in the genome of AAV and constitutes the capsid of AAV. The wild-type AAV genome encodes three capsid proteins, and there are VP1, VP2 and VP3. As used herein, the capsid protein includes VP1, VP2 and VP3. The capsid protein is sometimes referred to as the "Cap protein".

As used herein, the term "several" in the context of substitution, deletion, insertion and/or addition of amino acids means, for example, 2, 3, 4, 5, 6, 7, 8 or 9 amino acids depending on the length of a reference amino acid sequence.

As used herein, the term "specific expression" does not mean that the expression product is expressed only in a particular tissue or cell type, but rather that the expression is substantially restricted to a particular tissue or cell type.

### (1) Nucleic acid encoding an AAV capsid protein mutant

The nucleic acid of the present invention encodes an AAV capsid protein mutant which comprises a peptide comprising an amino acid sequence selected from the group consisting of SEQ ID NOs: 1-7 or a peptide comprising an amino acid sequence that differs from an amino acid sequence selected from the group consisting of SEQ ID NOs: 1-7 by substitution, deletion, insertion and/or addition of one or several amino acids. Preferably, the nucleic acid of the present invention encodes an AAV capsid protein mutant that comprises a peptide comprising an amino acid sequence selected from the group consisting of SEQ ID NOs: 1-7. The peptide comprising an amino acid sequence that differs from an amino acid sequence shown by the above-mentioned SEQ ID NOs by substitution, deletion, insertion and/or addition of one or several amino acids, when contained in an AAV capsid protein, retains the cell tropism of an AAV capsid protein mutant that comprises a peptide comprising an amino acid sequence shown by the above-mentioned SEQ ID NOs. In other words, the number of amino acids to be substituted, deleted, inserted and/or added in an amino acid sequence shown by the above-mentioned SEQ ID NOs is not limited as long as the cell tropism that a peptide comprising an amino acid sequence shown by the above-mentioned SEQ ID NOs confers to the AAV capsid protein mutant comprising the peptide is retained. For example, 1 to 5, preferably 1 to 4, more preferably 1, 2 or 3 amino acids may be substituted, deleted and/or inserted. For example, 1 to 9, preferably 1 to 8, more preferably 1 to 7, still more preferably 1 to 6, still more preferably 1 to 5, still more preferably 1, 2, 3 or 4 amino acids may be added.

For example, the peptide to be contained in the AAV capsid protein mutant may be a peptide comprising an amino acid sequence having at least 70%, at least 75%, at least 80%, at least 85%, or at least 90% identity with an amino acid sequence selected from the group consisting of SEQ ID NO: 1 to SEQ ID NO: 7. The peptide comprising an amino acid sequence having at least 70%, at least 75%, at least 80%, at least 85%, or at least 90% identity with an amino acid sequence shown by the above-mentioned SEQ ID NOs, when contained in an AAV capsid protein, retains the cell tropism of an AAV capsid protein mutant comprising a peptide comprising an amino acid sequence shown by the above-mentioned SEQ ID NOs.

The AAV capsid protein mutant encoded by the nucleic acid of the present invention can be prepared by inserting the peptide into an AAV capsid protein of any wild-type AAV, such as AAV type 1 (AAV1), AAV type 2 (AAV2), AAV type 3 (AAV3A, AAV3B etc.), AAV type 4 (AAV4), AAV type 5 (AAV5), AAV type 6 (AAV6), AAV type 7 (AAV7), AAV type 8 (AAV8), AAV type 9 (AAV9), AAV type 10 (AAV10), AAV type 11 (AAV11), AAV type 12 (AAV12), AAV type 13 (AAV13), avian AAV, bovine AAV, canine AAV, equine AAV, or ovine AAV, or replacing a part of the amino acid sequence of the AAV capsid protein with the peptide (in other words, by making the AAV capsid protein comprise the peptide). In the present invention, a capsid protein of AAV2 is preferably used. The amino acid sequence of the VP1 capsid protein of wild-type AAV2 is shown in SEQ ID NO: 12 (GenBank: AAC03780.1).

The AAV capsid protein mutant encoded by the nucleic acid of the present invention may be a protein comprising substitution, deletion, insertion and/or addition of one or more, for example, one to several amino acids as well as the above-mentioned peptide in the wild-type AAV capsid protein. The "protein comprising substitution, deletion, insertion and/or addition of one or more amino acids as well as the above-mentioned peptide" retains the properties of the original protein, for example, the cell tropism of the AAV capsid protein mutant conferred by the above-mentioned peptide, the capsid-forming ability, the function of capsid protein (for example, protection of viral genome, uncoating after entry into host cells) and the like. In the present invention, for example, an AAV2 capsid protein in which asparagine at position 587 is replaced with glutamine is used. The amino acid sequence of the AAV2 VP1 capsid protein comprising glutamine at position 587 is shown in SEQ ID NO:13.

Furthermore, the amino acid sequence of the AAV capsid protein mutant encoded by the nucleic acid of the present invention has 80% or more, preferably 90% or more, and particularly preferably 95% or more identity to the amino acid sequence of the wild-type AAV capsid protein.

Further, a spacer may be added to the N terminal and/or C terminal of the peptide to be contained in the AAV capsid protein. The spacer preferably consists of 1 to 5 amino acid residues. The amino acid residues constituting the spacer are not particularly limited. For example, the spacer may comprise an amino acid selected from the group consisting of glycine, alanine and serine. A single amino acid residue of glycine is suitable as a spacer added to the N-terminus. A single amino acid residue of alanine is suitable as a spacer added to the C-terminus.

As the AAV capsid protein for comprising the peptide, AAV VP1, VP2 or VP3 may be used. Only any one of VP1, VP2 and VP3 may be made to comprise the peptide, or all of VP1, VP2 and VP3 may be made to comprise the peptide. Furthermore, two capsid proteins such as VP1 and VP2, VP2 and VP3, or VP1 and VP3 may be made to comprise the peptide. VP1 to VP3 are encoded by the cap gene region in the AAV genome. In one embodiment of the present invention, a region shared by VP1 to VP3 is made to comprise the peptide so that a mutation can be introduced into all of VP1 to VP3. In another embodiment of the present invention, a gene encoding VP1, VP2 or VP3 is prepared separately from the cap gene region of AAV, and a mutation is introduced into the gene. In this case, a treatment that inhibits a wild-type capsid protein corresponding to a capsid protein encoded by the gene into which a mutation has been introduced from being expressed from the cap gene region of AAV may be performed.

In the case where AAV2 VP1 is used, the AAV capsid protein mutant encoded by the nucleic acid of the present invention preferably comprises the peptide at a position between amino acid number 588 and amino acid number 589. The amino acid number 588 of AAV2 VP1 is arginine. The amino acid number 589 of AAV2 VP1 is glutamine. The amino acid number 588 of AAV2 VP1 corresponds to the amino acid number 451 of AAV2 VP2 and the amino acid number 386 of AAV2 VP3. In the case where a capsid protein of AAV serotypes and clades other than AAV2 is used as the AAV capsid protein, the AAV capsid protein is made to comprise the peptide between amino acids corresponding to amino acid numbers 588 and 589 of AAV2 VP1. A person skilled in the art can easily identify an amino acid of a capsid protein of AAV serotypes and clades other than AAV2 which corresponds to the amino acid at amino acid number 588 of AAV2 VP1. For example, see an alignment of amino acid sequences of VP1 shown in Gao et al., Proc. Natl. Acad. Sci. USA, Vol.99, No.18, pp. 11854-11859, 2002. For example, the amino acid number 588 of AAV2 VP1 corresponds to the amino acid number 589 of AAV1, the amino acid number 590 of AAV7, and the amino acid number 591 of AAV8.

In the present invention, an AAV capsid protein mutant (e.g., SEQ ID NO: 14) containing a spacer(s) and DKAWLVG (SEQ ID NO: 1) between amino acid positions 588 and 589 of the AAV2 VP1 capsid protein in which glutamine is present at position 587 is preferably used.

The nucleic acid of the present invention may be operably linked to a suitable control sequence. Examples of the control sequence include a promoter sequence, a polyadenylation signal, a transcription termination sequence, an upstream regulatory domain, a replication origin, an internal ribosomal entry site (IRES), and an enhancer. Examples of the promoter sequence include an inducible promoter sequence, and a constitutive promoter sequence. The control sequence may be an endogenous or exogenous sequence of AAV from which the capsid protein originates, a native sequence, or an artificially designed sequence. The present invention also includes such a recombinant DNA capable of expressing the AAV capsid protein mutant.

The recombinant DNA of the present invention is useful for delivering the nucleic acid of the present invention to cells in vitro, ex vivo or in vivo and imparting the ability to express the AAV capsid protein mutant to the cells. Then, the cell to which the nucleic acid of the present invention is delivered is useful for producing AAV particles. The recombinant DNA can deliver or introduce the nucleic acid of the present invention into various cells, preferably animal cells, more preferably mammal cells.

In the present invention, the recombinant DNA of the present invention can be prepared by making a DNA used as a vector retain the nucleic acid of the present invention. For example, a plasmid DNA, a phage DNA, a transposon, a cosmid DNA, an episomal DNA, or a viral genome can be used.

### (2) Cell containing the nucleic acid of the present invention

The present invention also provides a cell comprising the nucleic acid of the present invention, specifically an isolated host cell containing the recombinant DNA as described in above (1). An isolated cell is, for example, a cell line maintained *in vitro.* The host cell of the present invention is useful for production of the AAV particle of the present invention, as explained below. When the host cell of the present invention is used for producing AAV particles, the host cell may be referred to as a "packaging cell" or "producer cell". The host cell of the present invention may comprise the recombinant DNA of the present invention as described in above (1) integrated into the genome, or retain the recombinant DNA in the cell so as to transiently express the AAV capsid protein mutant.

Introduction of the recombinant DNA of the present invention into a host cell can be performed by a known method. For example, electroporation, calcium phosphate precipitation, direct microinjection into cells, liposome-mediated gene transfection, or nucleic acid delivery using a high-speed particle gun can be used. When a viral vector is used, an infection method suitable for the vector may be selected. By use of such an established technique, the recombinant DNA of the present invention is introduced stably into a chromosome of a host cell or transiently into a cytoplasm of a host cell. For stable transformation, a selectable marker, for example, a well-known selectable marker such as a neomycin resistance gene (encoding neomycin phosphotransferase), or a hygromycin B resistance gene (encoding aminoglycoside phosphotransferase (APH)) may be linked to the recombinant DNA of the present invention.

As the host cell, various cells, for example, mammal cells including mouse cells and primate cells (for example, human cells) or insect cells can be used. Suitable examples of mammal cells include, but are not limited to, primary cells and cell lines. Examples of suitable cell lines include 293 cells, COS cells, HeLa cells, Vero cells, 3T3 mouse fibroblasts, C3H10T1/2 fibroblasts, CHO cells, and cells derived from them.

### (3) AAV particle comprising an AAV capsid protein mutant encoded by the nucleic acid of the present invention

The AAV particle of the present invention is an AAV particle comprising an AAV capsid protein mutant comprising a peptide comprising an amino acid sequence selected from the group consisting of SEQ ID NO: 1 to SEQ ID NO: 7 or a peptide comprising an amino acid sequence that differs from an amino acid sequence selected from the group consisting of SEQ ID NO: 1 to SEQ ID NO: 7 by substitution, deletion, insertion and/or addition of one or several amino acids, encoded by the nucleic acid as described in above (1). The AAV particle of the present invention can be produced from the host cell described in above (2). The AAV particle of the present invention has infectivity for the inner ear, and is useful for gene introduction into the inner ear. The inner ear contains cells such as hair cells, supporting cells, nerve cells, and fibrocytes. The gene introduced by the AAV particle of the present invention is strongly expressed in the above-mentioned tissues, organs and cells.

For production of the AAV particle, a cell comprising some elements necessary for production of AAV particles can be used as a packaging cell. The first element is a vector genome (also referred to as an expression vector) for a recombinant AAV which may be replicated in a host cell and packaged in an AAV particle. The recombinant AAV vector genome comprises a heterologous polynucleotide of interest, and AAV inverted terminal repeat (ITR) sequences located on each side, i.e., 5'- and 3'-sides of the heterologous polynucleotide of interest. As used herein, the term "heterologous polynucleotide" refers to a polynucleotide heterologous to AAV. The heterologous polynucleotide of interest may have a control sequence for the expression. The nucleotide sequences of ITR sequences are known. For AAV2-ITR sequences, for example, see Human Gene Therapy, Vol.5, pp. 793-801, 1994. As the AAV ITR sequences, ITR sequences derived from any of various AAV serotypes including AAV1, AAV2, AAV3, AAV4, AAV5, AAV7 and the like can be used. The ITR sequences used in the present invention may be derived from a wild-type AAV or may be altered by insertion, deletion or substitution of a nucleotide(s). The ITR sequences enable replication of the recombinant AAV vector genome in the presence of Rep protein, and enable incorporation of the vector genome into a capsid particle in the formation of an AAV particle.

The size of the heterologous polynucleotide of interest which can be harbored inside the AAV particle of the present invention is generally less than about 5 kilobases (kb). The heterologous polynucleotide of interest may be, for example, a gene that causes a genetic disease, a gene involved in cell differentiation and/or proliferation, a gene encoding a protein having a desired biological or therapeutic activity (for example, antimicrobial, antiviral, or antitumor activity), a desired nucleotide sequence encoding RNA that inhibits or decreases production of a harmful or undesired protein, a nucleotide sequence encoding an antigenic protein, a genome editing-related gene, a reporter gene, or a selectable marker gene. The heterologous polynucleotide of interest can be appropriately selected according to purposes.

Examples of the genetic diseases include hereditary hearing loss, hereditary skin diseases, congenital palmoplantar keratoderma, congenital ichthyosis, and hereditary retinal diseases. Examples of genes that cause the genetic diseases include the connexin 26 (GJB2) gene, the pendrin (SLC26A4) gene, the TMC1 gene, the TMC2 gene, the voltage-gated potassium channel subfamily Q member 4 (KCNQ4) gene, the cadherin 23 (CDH23) gene, the protocadherin 15 (PCDH15) gene, and the otoferlin (OTOF) gene.

Examples of the gene involved in cell differentiation and/or proliferation include genes involved in the differentiation and/or proliferation of inner ear cells, and specifically, but are not limited to, the protein atonal homolog 1 (Atoh1) gene, the cyclindependent kinase inhibitor 1B (CDKN1B) gene, and the basic helix-loop-helix transcription factor (Hes1) gene.

Further, examples of the heterologous polynucleotides of interest that can be harbored inside the AAV particle of the present invention include ACTG1, ADCY1, ADGRV1, AIFM1, BDP1, BSND, BTD, CABP2, CCDC50, CD164, CDC14A, CEACAM16, CIB2, CLDN14, CLIC5, CLRN1, COCH, COL11A1, COL11A2, COL2A1, COL4A3, COL4A4, COL4A5, COL4A6, COL9A1, COL9A2, COL9A3, DCDC2, DIAPH1, DMXL2, DSPP, EDN3, EDNRB, ELMOD3, EPS8, EPS8L2, ESPN, ESRRB, EYA1, EYA4, FAM189A2, GIPC3, GJB3, GJB6, GPSM2, GRHL2, GRXCR1, GRXCR2, GSDME, HARS1, HGF, HOMER2, ILDR1, KARS1, KCNE1, KCNQ1, LHFPL5, LOXHD1, LRTOMT, MARVELD2, MCM2, MET, MIR96, MITF, MSRB3, MT-CO1, MT-RNR1, MT-TS1, MYH14, MYH9, MYO15A, MYO1A, MYO3A, MYO6, MYO7A, MYO7A, NARS2, NF2, OSBPL2, OTOA, OTOG, OTOGL, P2RX2, PAX3, PEX7, PHYH, PJVK, PNPT1, POU3F4, POU4F3, PRPS1, PTPRQ, RDX, RIPOR2, ROR1, S1PR2, SERPINB6, SIX1, SIX5, SLC17A8, SLC22A4, SLC26A5, SMPX, SOX10, STRC, SYNE4, TBC1D24, TECTA, TIMM8A, TJP2, TMEM132E, TMIE, TMPRSS3, TPRN, TRIO, TSPEAR, USH1C, USH1G, USH2A, WBP2, WFS1, and WHRN.

Further, examples of the reporter gene include nucleic acids encoding fluorescent polypeptides (GFP, RFP, AcGFP, etc.) or active fragments thereof.

In some embodiments, the heterologous polynucleotide of interest is operably linked to a constitutive promoter. In other embodiments, the heterologous polynucleotide of interest is operably linked to an inducible promoter. In some cases, the heterologous polynucleotide of interest is functionally linked to a tissue-specific or cell type-specific regulatory element. For example, the heterologous polynucleotide of interest desired to be expressed in inner ear hair cells is operably linked to a regulatory element that confers selective expression of a nucleic acid in inner ear hair cells (Hum Mol Genet. 2001 Jul 15;10(15):1581-9).

In one embodiment of the present invention, the recombinant AAV vector genome lacks the cap gene region and/or the rep gene region. In this embodiment, an AAV particle into which the recombinant AAV vector genome is packaged is not replicated alone to form an AAV particle again in an infected cell.

The second element necessary for production of AAV particles is a construct that provides proteins encoded in the wild-type AAV. The construct encodes AAV-derived genes providing AAV gene products required for formation of AAV particles. In other words, the construct comprises one or both of the major AAV ORFs, coding regions of the rep gene region and cap gene region. For production of the AAV particle of the present invention, at least a nucleic acid encoding an AAV capsid protein mutant comprising a peptide comprising an amino acid sequence selected from the group consisting of SEQ ID NO: 1 to SEQ ID NO: 7 is used as the cap gene. The host cell of the present invention described in above (2) which is capable of expressing the mutant can be used for production of the AAV particle. The AAV particle has a shell composed of many capsid proteins. All of the capsid proteins may be mutants, or a part of the capsid proteins may be mutants and the others may be wild-type capsid proteins. The AAV particle of the present invention may comprise one kind of a capsid protein mutant or plural kinds of a capsid protein mutants.

The rep gene-coding region of AAV includes genes encoding replication proteins Rep78, Rep68, Rep52 and Rep40. These Rep expression products are shown to possess many functions, including recognition, binding and nicking of the AAV genomic DNA replication origin, DNA helicase activity, and modulation of transcription from AAV-derived promoters.

The third element necessary for production of AAV particles is helper virus functions (also referred to as accessary functions) for AAV replication. For introduction of the helper functions, an adenovirus is generally used. However, other viruses such as herpes simplex virus type-1 or type-2, and vaccinia virus can be also used. When a virus is used, a host cell is infected with the virus as a helper virus. For example, since expression of adenovirus early genes is only required for packaging of AAV particles, an adenovirus that does not reveal expression of late genes may be used. An adenovirus mutant lacking late gene expression (for example, ts100K or ts149 adenovirus variant) can be also used. A nucleic acid construct that provides helper virus functions can be also prepared by use of nucleic acids necessary for the helper virus functions isolated from a helper virus, and then can be introduced into a host cell. The construct that provides the helper virus functions comprises a nucleotide sequence providing one or plural helper virus functions, and is provided to a host cell in the form of a plasmid, phage, transposon, cosmid, or other viruses.

For production of AAV particles, (a) a step of introducing the first element, the recombinant AAV vector genome into a host cell, (b) a step of introducing the second element, the construct that provides AAV helper functions into the host cell, and (c) a step of introducing the third element, the helper virus functions into the host cell are performed. These steps may be performed at the same time, or may be performed in order. The order of steps (a) to (c) may be any order. When the first to third elements are introduced into a host cell, the rep gene-expression products excise and replicate the recombinant vector genome. The capsid proteins expressed form a capsid, and the recombinant vector genome is packaged in the capsid to produce an AAV particle. When the host cell expresses an AAV capsid protein mutant, the shell of the AAV particle produced comprises the AAV capsid protein mutant.

The AAV particle can be isolated and purified from a culture supernatant or a lysate of the host cell by various purification methods such as CsCl density-gradient centrifugation. When a virus is used in above-described step (c), for example, a step of separating the AAV particle from the helper virus on the basis of their size may be added. The AAV particle can be also separated from the helper virus on the basis of a difference in affinity for heparin. Furthermore, the remaining helper viruses can be inactivated by known methods. For example, adenoviruses can be inactivated by heating at about 60°C, for example, for 20 minutes or more. Since AAV particles are very stable to heat, the above-described treatment is effective for selective removal of adenoviruses used as the helper virus.

### (4) Method of producing a gene-transduced cell of the present invention

The AAV particles of the present invention obtained by (3) above are used to deliver a heterologous polynucleotide of interest to cells (target cells) for gene therapy or other purposes. The target cells are animal cells, preferably mammalian cells, more preferably human cells. The cells (target cells) to which AAV particles deliver a heterologous polynucleotide preferably include, but are not limited to, cells of the inner ear (hair cells, supporting cells, nerve cells, and fibrocytes). For example, epithelial cells, stromal cells, lens cells, or cancer cells can also be the target cells. The epithelial cells include inner ear epithelial cells, intestinal epithelial cells, retinal cells, skin cells, kidney cells, airway epithelial cells, nerve cells, and uterine cells. Furthermore, cells that have gap junctions or express connexin molecules that are components of gap junctions can also be the target cells.

The AAV particle is generally introduced into a cell *in vivo* or *in vitro.* For in vitro introduction, the AAV particle is brought into contact with a cell obtained from a living body. Then, the cell can be also administered to a living body. For administration of the cell to a living body, the cell can be formulated as a pharmaceutical composition, and various techniques such as intramuscular, intravenous, subcutaneous and intraperitoneal administration can be used. For *in vivo* transduction, the AAV particle is formulated as a pharmaceutical composition, and in general, administered parenterally (for example, administered via an intramuscular, subcutaneous, intratumor, transdermal, or intraspinal route). The pharmaceutical composition comprising the AAV particle may contain a pharmaceutically acceptable carrier and, as necessary, other drug, medicine, stabilizer, carrier, adjuvant, diluent, and the like. The dosage of the pharmaceutical composition can be appropriately determined depending on administration conditions such as a subject of administration, a route of administration, and a dosage form.

The pharmaceutical composition comprising the AAV particle of the present invention can be used to deliver a heterologous polynucleotide of interest, for example a polynucleotide effective in the prevention or treatment of disease, to a cell, tissue, or organ which the AAV particle has tropism for, preferably to an inner ear tissue or a cell of the inner ear (e.g., hair cells, supporting cells, nerve cells, fibrocytes). In other words, the pharmaceutical composition is useful as a preventive or therapeutic agent for disease. Furthermore, a method for preventing or treating a disease, the method comprising using the pharmaceutical composition is also provided as an aspect of the present invention.

Therefore, one aspect of the present invention provides a pharmaceutical composition for preventing or treating a disease, comprising cells (gene-transduced cells) into which a polynucleotide effective for preventing or treating the disease has been introduced, the cells being produced by contacting the AAV particles of the present invention carrying the polynucleotide with cells *ex vivo.* In yet another aspect, there is provided a pharmaceutical composition for preventing or treating a disease, comprising the AAV particles of the present invention loaded with a polynucleotide effective for preventing or treating the disease. In a further aspect, there is provided a method for preventing or treating a disease, the method comprising administering the pharmaceutical composition, the gene-transduced cell, or the AAV particle of the present invention loaded with a polynucleotide effective for preventing or treating the disease, to a subject. Examples of the disease include, but are not limited to, inner ear diseases, skin diseases, eye diseases, head and neck diseases, and mammary gland diseases, and preferred examples include hereditary hearing loss, hereditary skin diseases, congenital palmoplantar keratosis, congenital ichthyosis, hereditary retinal diseases, head and neck cancer, and breast cancer. The polynucleotide effective for preventing or treating the above-mentioned disease is appropriately selected depending on the disease to be prevented or treated. Examples of the polynucleotide include, but are not limited to, a gene that causes a genetic disease, a gene involved in cell differentiation and/or proliferation, a gene encoding a protein having a desired biological or therapeutic activity (for example, antimicrobial, antiviral, or antitumor activity), a desired nucleotide sequence encoding RNA that inhibits or decreases production of a harmful or undesired protein, a nucleotide sequence encoding an antigenic protein, and a genome editing-related gene, as mentioned in (3) above.

### EXAMPLES

Hereinafter, the present invention is explained with reference to Examples which the present invention is not particularly limited to.

### Example 1: Preparation of AAV2 plasmid library (round 0)

Plasmid pAV1 (ATCC number: 37215) was extracted from a host *Escherichia coli* HB101 competent cell (manufactured by Takara Bio Inc.). AAV2 genomic DNA (about 4.7 kb) was excised from the plasmid pAV1 using restriction enzyme BglII (manufactured by Takara Bio Inc.). The genomic DNA was inserted into pUC118 BamHI/BAP (manufactured by Takara Bio Inc.). The resulting plasmid was designated AAV2WG/pUC118.

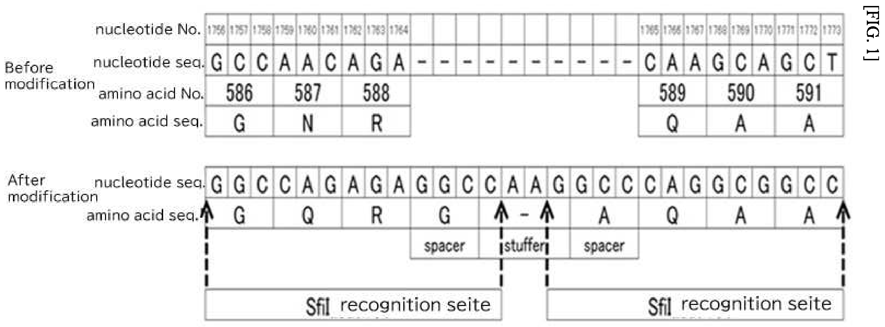

AAV2WG/pUC118 was digested with restriction enzyme ScaI (manufactured by Takara Bio Inc.) to obtain an approximately 0.8 kb fragment containing nucleotides from positions 1190 to 2017 of the Cap gene. The fragment was inserted into pUC118 HincII/BAP (manufactured by Takara Bio Inc.). The resulting plasmid was designated Cap-ScaI/pUC118. The nucleotide sequence of the Cap gene contained in Cap-ScaI/pUC118 was modified by PCR in the following three ways: (1) conversion of the nucleotide sequence AAC(587N) from positions 1759 to 1761 to CAG(587Q), (2) insertion of a total of 10 nucleotides consisting of GGC as a spacer, CAAG as a stuffer and GCC as another spacer between nucleotide positions 1764 and 1765, and (3) conversion of the nucleotide sequence CAA(589Q) GCA(590A) GCT(591A) from positions 1765 to 1773 to CAG(589Q) GCG(590A) GCC(591A), wherein the letters in parentheses indicate an amino acid number and an encoded amino acid. This modification resulted in the formation of a first SfiI recognition site, a spacer, a stuffer, a spacer, and a second SfiI recognition site. In addition, asparagine (N) at position 587 in the capsid encoded by the Cap gene was substituted with glutamine (Q). The nucleotide sequence of the Cap gene before and after the modification at positions 1756 to 1773 and the amino acid sequence of the Cap protein before and after the modification at positions 586 to 591 are shown in FIG. 1. The modified plasmid was designated Cap-ScaI-S4/pUC118. The Cap gene portion of Cap-Scal-S4/pUC118 was amplified by PCR, and an amplification product of about 0.8 kb was obtained. The amplified product was used as an insert DNA for in-fusion cloning.

The ScaI recognition site in the ampicillin resistance gene and the SfiI recognition site in the Rep gene of AAV2WG/pUC118 were modified by PCR to sequences that were not recognized by the respective restriction enzymes. More specifically, for the ScaI recognition site, the nucleotide sequence GAG(E) from positions 304 to 306 of the ampicillin resistance gene was converted to GAA(E). On the other hand, for the SfiI recognition site, the nucleotide sequence GCC(A) from positions 217 to 219 of the Rep gene was converted to GCA(A). The thus obtained plasmid was digested with Scal (manufactured by Takara Bio Inc.) to obtain a linear vector. This vector contained a deletion of approximately 0.8 kb in the Cap gene, and was used as a linear vector for in-fusion cloning.

Next, using In-Fusion (registered trademark) HD Cloning Kit (manufactured by Takara Bio Inc.) and Cloning Enhancer (manufactured by Takara Bio Inc.), an insert DNA was inserted into the linear vector. The resulting plasmid was designatedAAV2WG-Cap-ScaI-S4/pUC118Sx.

An oligo-DNA (SEQ ID NO: 8) containing a nucleotide sequence encoding a random peptide of 7 amino acids was artificially synthesized. The oligo DNA, a primer (SEQ ID NO: 9), and a klenow fragment (manufactured by Takara Bio Inc.) were mixed and reacted at 37°C for 3 hours. Through this reaction, a double-stranded DNA was produced using the oligo DNA as a template. The double-stranded DNA was purified using a Nucleotide Removal Kit (manufactured by Qiagen) and then digested with restriction enzyme BglI (manufactured by Takara Bio Inc.). The DNA was inserted into AAV2WG-Cap-ScaI-S4/pUC118Sx which had been previously digested with SfiI, using DNA Ligation Kit <Mighty Mix> (manufactured by Takara Bio Inc.). The resulting plasmid was designated AAV2WG-RPL/pUC118Sx, and used as the AAV2 plasmid library (round 0).

### Example 2: Preparation of AAV2 library containing random peptides

### (1) Seeding of 293T cell

In DMEM (manufactured by Sigma) containing 10% FBS and a 1% penicillin/streptomycin solution, 293T cells were suspended and seeded into CellBIND surface HyperFlask cell culture vessels (manufactured by Corning) at 3 x 10⁷ cells/flask. The 293T cells were cultured in a CO₂ incubator at 37°C for 72 hours.

### (2) Introduction of plasmids into 293T cell

The AAV2WG-RPL/pUC118Sx obtained in Example 1 and pHelper Vector (manufactured by Takara Bio Inc.) were transfected into 293T cells using PEIpro (registered trademark) (manufactured by Polyplus transfection Inc.). The 293T cells were cultured in a CO₂ incubator at 37°C for 72 hours.

### (3) Recovery of AAV2 library containing random peptides

In a solution containing a surfactant, 293T cells were suspended and then solubilized. The suspension was collected in a 500 mL centrifuge tube and centrifuged to collect a supernatant.

### (4) Measurement of viral genome quantity by real-time PCR

The viral genome quantity contained in the supernatant collected in Example 2-(3) was measured using AAVpro (registered trademark) Titration Kit Ver. 2 (manufactured by Takara Bio Inc.).

### Example 3: Purification of AAV2 library containing random peptides

### (1) Affinity Chromatography

The supernatant collected in Example 2-(3) was filtered using a 0.45 µm bottle-top filter (manufactured by Corning) and then applied to AVB Sepharose High Performance (manufactured by Cytiva). The eluate was separated into 1 mL fractions, and the amount of viral genome contained in each fraction was measured using the same method as in Example 2-(4). Each fraction was subjected to protein quantification by A280 measurement. As a result, fractions enriched in AAV vectors were found.

### (2) Cesium chloride density gradient centrifugation

After mixing the fractions enriched in AAV vectors obtained in Example 3-(1), a cesium chloride solution was added to adjust the refractive index to 1.371. The mixture was dispensed into ultracentrifuge 13PA tubes (manufactured by Eppendorf Himac Technologies) and centrifuged at 34,000 rpm and 21°C for 42 hours. After centrifugation, the supernatant was fractionated into 0.5 mL fractions from the top of the tube. Using the same method as in Example 3-(1), fractions enriched in AAV vectors were found.

### (3) Dialysis

The fractions enriched in AAV vectors obtained in Example 3-(2) were mixed and then subjected to a Slide-A-lyzer dialysis cassette (manufactured by Thermo Scientific). The dialysis cassette was immersed in 1 L of a phosphate buffered saline (PBS), and dialysis was carried out at 4°C for 2 hours or more three times. Next, the dialysis cassette was immersed in 1 L of a PBS containing 0.001% Pluronic F-68 (manufactured by Thermo Scientific), 5% sorbitol and 200 mM NaCl, and dialyzed at 4°C for 3 hours. A solution was recovered from the dialysis cassette, filtered using a 0.22 µm filter (manufactured by Millipore), and then stored at -80°C until use as an "AAV2 library containing random peptides". The viral genome quantity was measured using the same method as in Example 2-(4).

### Example 4 Screening of AAV2 library containing random peptides

### (1) In vitro screening using mouse cochlear organ culture tissue

Mouse cochlear organ culture tissue was prepared using a method as described in Yodosha, Experimental Medicine Special Edition, "Definitive Edition; Experimental Guide for Gene Transfer Using Viral Vectors", pages 159-166 (November 2020), Kazusaku Kamiya. Specifically, four-day-old mice were euthanized under anesthesia, and the cochlea was removed from the temporal bone. The modiolus was removed from the cochlea, and the stria vascularis and lateral wall were peeled off to extract the cochlear epithelium. The cochlear epithelium was transferred to an 8-well chamber slide and cultured overnight (5% CO₂, 37°C) in 100 µL of a DMEM medium (containing high glucose, sodium pyruvate, GlutaMAX^{™} Supplement, fetal bovine serum, N2 Supplement, and penicillin G potassium). Thereafter, the medium was replaced with a medium containing 4 µL of the "AAV2 library containing random peptides" obtained in Example 3-(3) and 96 µL of the DMEM medium, thereby infecting the cochlear epithelium with AAV (final concentration: 2.8×10¹¹ vg/mL). After further culturing for 5 days, the cochlear epithelium was trypsinized to separate cells. The separated cells were labeled with rabbit anti-Myo7a antibody and PE-labeled anti-rabbit antibody. Myo7a-positive cells and Myo7a-negative cells were separated using a flow cytometer (BD FACSAria^{™}, manufactured by BD Biosciences). Myo7a is a marker for hair cells.

### (2) Recloning of random peptide sequences by PCR

A genomic DNA was extracted from the Myo7a-negative cells isolated in Example 4-(1) using NucleoSpin (registered name) Tissue (manufactured by Machlinergel). Using the genomic DNA as a template, DNAs encoding the random peptide sequences were amplified using PrimeSTAR (registered trademark) Max DNA Polymerase (manufactured by Takara Bio Inc.). As primers, a forward primer (SEQ ID NO: 10) and a reverse primer (SEQ ID NO: 11) were used. PCR was carried out under the conditions of preheating at 94°C for 1 minute, followed by 35 cycles of 98°C for 10 seconds, 55°C for 15 seconds, and 72°C for 30 seconds. PCR products were electrophoresed and then purified using NucleoSpin (registered trademark) Gel and PCR Clean-up (manufactured by Machlinergel). The purified PCR product was recloned into the linear vector prepared in Example 1. The resulting plasmids were used as an AAV2 plasmid library (round 1).

### (3) Preparation and purification of AAV2 library containing random peptides

Using the AAV2 plasmid library (round 1) obtained in Example 4-(2), an AAV2 library containing random peptides was prepared and purified in the same manner as in Examples 2 and 3.

### (4) In vivo screening by direct administration to inner ear organ of adult mouse

The AAV2 library containing random peptides prepared in Example 4-(3) was directly administered to the inner ear organ of an adult mouse. Administration to the inner ear organ was performed using a method as described in Yodosha, Experimental Medicine Special Edition, "Definitive Edition; Gene Transfer Experiment Guide Using Viral Vectors", pages 159-166 (November 2020), Kazusaku Kamiya. Specifically, after an incision was made behind the ear of an adult mouse (3-4 weeks old) under anesthesia, a small hole was made in each of the posterior semicircular canal and the lateral semicircular canal using a dental drill. A capillary tube was inserted into one of the small holes, and about 10 µL (about 2 to 10×10¹² vg/mL) of the AAV2 library containing random peptides prepared in Example 4-(3) was injected at a rate of 3 µL/min. Three weeks after administration, the mouse was euthanized and the cochlea was removed. Under a stereomicroscope, the organ of Corti and the lateral wall were removed from the cochlea, and then treated with trypsin to separate cells. The separated cells were labeled with a rabbit anti-Cx26 antibody and a PE-labeled anti-rabbit antibody, and then CX26-positive cells and CX26-negative cells were separated using a flow cytometer (BD FACSAria^{™}, manufactured by BD Biosciences).

An AAV2 plasmid library (round 2) was prepared from a genomic DNA of the CX26-positive cells derived from the organ of Corti in the same manner as in Example 4-(2). Using this library (round 2), an AAV2 library containing random peptides prepared and purified by the same method as in Example 4-(3) was directly administered to the inner ear organ of an adult mouse. From this mouse, an AAV2 plasmid library (round 3) was prepared in the same manner as above.

### (5) Next-generation sequencing (NGS) analysis of random peptide regions

NGS analysis was performed using Miseq (manufactured by Illumina) on the random peptide regions of the AAV2 plasmid libraries (rounds 1 to 3) to clarify the frequency of occurrence of random peptides. Results of the AAV2 plasmid library (round 3) derived from the organ of Corti are shown in Table 1. Results of the AAV2 plasmid library (round 3) derived from the lateral wall are shown in Table 2.

**[Table 1]**

| Sequence | ID | Read | Frequency |
|---|---|---|---|
| GGVAKPP | SEQ ID NO:4 | 3491 | 8.92% |
| GPTPRQG | SEQ ID NO:3 | 2537 | 6.48% |
| DGLQRRE | SEQ ID NO:2 | 2221 | 5.68% |
| VKGEWLG | | 1697 | 4.34% |
| DKAWLVG | SEQ ID NO:1 | 1519 | 3.88% |
| ATGQGYS | | 1372 | 3.51% |
| MHGTVVG | | 1351 | 3.45% |
| GGSLCAG | | 1129 | 2.88% |
| VYQGGSG | | 1075 | 2.75% |
| EAGGRLG | | 978 | 2.50% |
| GTYPGAG | SEQ ID NO:5 | 977 | 2.50% |
| NEVARRE | SEQ ID NO:7 | 913 | 2.33% |
| MGPLGRE | | 901 | 2.30% |
| VSVTSGG | | 849 | 2.17% |
| QESWARG | | 811 | 2.07% |
| REGERRE | | 776 | 1.98% |
| DVPWSGA | | 758 | 1.94% |
| LVGYTGS | | 748 | 1.91% |
| YGGAGKE | | 675 | 1.72% |
| VLGAGRE | | 638 | 1.63% |
| TVREGGE | | 637 | 1.63% |
| AYAQQAP | SEQ ID NO:6 | 549 | 1.40% |
| QAYAGGG | | 547 | 1.40% |
| GGLVSQG | | 525 | 1.34% |
| VVSGMPP | | 520 | 1.33% |

**[Table 2]**

| Sequence | ID | Read | Frequency |
|---|---|---|---|
| GGVAKPP | SEQ ID NO:4 | 5488 | 8.66% |
| DGLQRRE | SEQ ID NO:2 | 4634 | 7.31% |
| GTYPGAG | SEQ ID NO:5 | 4494 | 7.09% |
| GPTPRQG | SEQ ID NO:3 | 4475 | 7.06% |
| DKAWLVG | SEQ ID NO:1 | 2221 | 3.51% |
| QTMGLRE | | 1934 | 3.05% |
| VSVTSGG | | 1496 | 2.36% |
| AGFGRPE | | 1469 | 2.32% |
| DVPWSGA | | 1314 | 2.07% |
| PVGPIRE | | 1089 | 1.72% |
| AGFQSGA | | 1078 | 1.70% |
| AYAQQAP | SEQ ID NO:6 | 1073 | 1.69% |
| AFAGGVG | | 1032 | 1.63% |
| VGGGRVD | | 941 | 1.49% |
| MGPLGRE | | 917 | 1.45% |
| YGGAGKE | | 894 | 1.41% |
| GVMRTGE | | 878 | 1.39% |
| NEVARRE | SEQ ID NO:7 | 876 | 1.38% |
| QAYAGGG | | 777 | 1.23% |
| DVGMRAS | | 766 | 1.21% |
| QESWARG | | 747 | 1.18% |
| ESIRVMG | | 745 | 1.18% |
| EGGMRAA | | 709 | 1.12% |
| TGMGSGA | | 691 | 1.09% |
| DLGMARM | | 687 | 1.08% |

As shown in Tables 1 and 2, it was found that AAV2 containing specific peptides accumulated. Among them, GGVAKPP (SEQ ID NO: 4), GPTPRQG (SEQ ID NO: 3), GTYPGAG (SEQ ID NO: 5), DGLQRRE (SEQ ID NO: 2), DKAWLVG (SEQ ID NO: 1), NEVARRE (SEQ ID NO: 7), and AYAQQAP (SEQ ID NO: 6) were found in both the organ of Corti (Table 1) and the lateral wall (Table 2). It is suggested that these peptide sequences may promote infection of the inner ear organs.

### Example 5: Evaluation of AAV vector carrying obtained peptide sequence

### (1) Construction of pRC plasmid

After spacer sequences of GGC and GCC were added respectively to the 5' end and the 3' end of nucleotide sequences encoding the peptide sequences (SEQ ID NO: 1 to SEQ ID NO: 7) obtained in Example 4-(5) , the nucleotide sequences were cloned into the cap gene (a position between amino acids corresponding amino acid numbers 588 and 589 of VP1) of pRC2-mi342 Vector (manufactured by Takara Bio Inc.). The seven pRC plasmids thus obtained are as follows. pRC2-mi342-GGVAKPP, pRC2-mi342-GPTPRQG, pRC2-mi342-GTYPGAG, pRC2-mi342-DGLQRRE, pRC2-mi342-DKAWLVG, pRC2-mi342-NEVARRE, pRC2-mi342-AYAQQAP.

### (2) Preparation and purification of AAV vector carrying obtained peptide sequence

Using PEIpro (manufactured by Polyplus), 293T cells were transfected with pAAV-CMV-AcGFP, pHelper Vector (manufactured by Takara Bio Inc.), and the pRC plasmid obtained in Example 5-(1). As a control, 293T cells were transfected with pRC2-mi342 Vector (manufactured by Takara Bio Inc.) carrying the wild-type AAV2 cap gene instead of the seven pRC plasmids. The transfected 293T cells were cultured in a CO₂ incubator at 37°C for 72 hours. The AAV vectors carrying the obtained peptide sequences were collected and then purified by the same method as in Example 2-(3).

### (3) Infection of mouse cochlear culture tissue

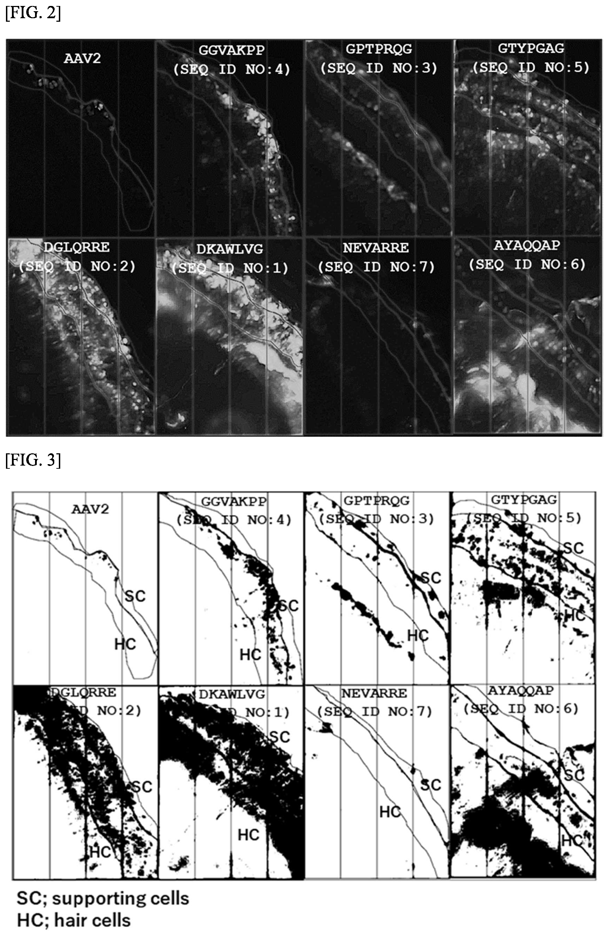

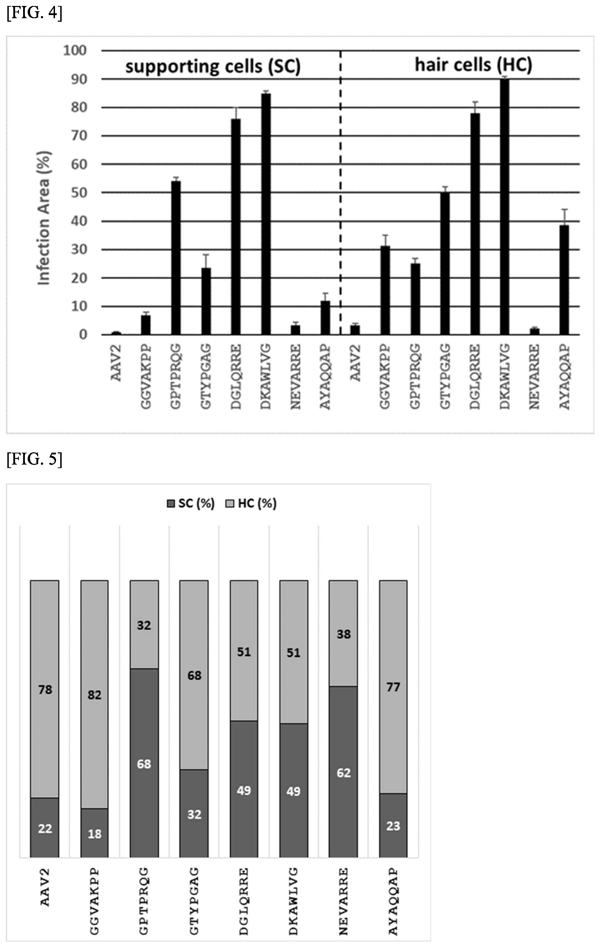

A mouse cochlear culture tissue was prepared using the same method as in Example 4-(1). Furthermore, the AAV vectors carrying the peptide sequences obtained in Example 5-(2) were added to the cultured mouse cochlea tissue at a concentration of 2×10 ¹¹ vg/mL, thereby infecting the tissue with the AAV vectors. The AAV vectors used for infection were wild-type AAV2 and AAV2 mutants with various peptide sequences (GGVAKPP, GPTPRQG, GTYPGAG, DGLQRRE, DKAWLVG, NEVARRE, and AYAQQAP). Three days after infection, the expression of AcGFP in the cultured mouse cochlea tissue was photographed using a fluorescence microscope (FIG. 2). These photographs were then converted into black and white images using image analysis software (ImageJ) (FIG. 3). In these images, regions mainly composed of supporting cells (SC) and regions mainly composed of hair cells (HC) were identified. The area infected with each AAV vector in each region (FIG. 4) and the infection ratios of SC and HC (FIG. 5), assuming the total AcGFP-expressing cells as 100%, were quantified.

As shown in FIG. 4 and 5, DKAWLVG (SEQ ID NO: 1) and DGLQRRE (SEQ ID NO: 2) had high infectivity in both SC and HC. Furthermore, GPTPRQG (SEQ ID NO: 3) was shown to have higher infectivity in SC than in HC.

### INDUSTRIAL APPLICABILITY

The present invention provides an AAV capsid protein mutant having infectivity for the inner ear, and a method for efficiently transferring genes into the inner ear.

### SEQUENCE LISTING FREE TEXT

SEQ ID NO:1: Peptide sequence DKAWLVG for AAV capsid protein mutant
SEQ ID NO:2: Peptide sequence DGLQRRE for AAV capsid protein mutant
SEQ ID NO:3: Peptide sequence GPTPRQG for AAV capsid protein mutant
SEQ ID NO:4: Peptide sequence GGVAKPP for AAV capsid protein mutant
SEQ ID NO:5: Peptide sequence GTYPGAG for AAV capsid protein mutant
SEQ ID NO:6: Peptide sequence AYAQQAP for AAV capsid protein mutant
SEQ ID NO:7: Peptide sequence NEVARRE for AAV capsid protein mutant
SEQ ID NO:8: DNA sequence coding random peptide
SEQ ID NO:9: Primer for synthesizing double strand DNA
SEQ ID NO:10: Forward primer for quantitation of AAV titer
SEQ ID NO:11: Reverse primer for quantitation of AAV titer
SEQ ID NO:12: Amino acid sequence of wild-type AAV2 VP1 capsid protein
SEQ ID NO:13: Amino acid sequence of AAV2(587Q) VP1 capsid protein
SEQ ID NO:14: Amino acid sequence of AAV2(587Q)-DKAWLVG VP1 capsid protein

## Claims

1. A nucleic acid encoding a mutant of an adeno-associated virus (AAV) capsid protein, the mutant comprising a peptide comprising an amino acid sequence selected from the group consisting of SEQ ID NOs: 1-7 or a peptide comprising an amino acid sequence that differs from an amino acid sequence selected from the group consisting of SEQ ID NOs: 1-7 by substitution, deletion, insertion and/or addition of one or several amino acids.

2. The nucleic acid according to claim 1, wherein the AAV capsid protein is derived from AAV2.

3. The nucleic acid according to claim 2, wherein the peptide is placed at a position between amino acid number 588 and amino acid number 589 in VP1 of AAV2.

4. A recombinant DNA comprising the nucleic acid according to any one of claims 1 to 3.

5. A cell comprising the nucleic acid according to any one of claims 1 to 3.

6. An AAV particle comprising a mutant of an AAV capsid protein which comprises a peptide comprising an amino acid sequence selected from the group consisting of SEQ ID NOs: 1-7 or a peptide comprising an amino acid sequence that differs from an amino acid sequence selected from the group consisting of SEQ ID NOs: 1-7 by substitution, deletion, insertion and/or addition of one or several amino acids.

7. The AAV particle according to claim 6, wherein the AAV capsid protein is derived from AAV2.

8. The AAV particle according to claim 7, wherein the peptide is placed at a position between amino acid number 588 and amino acid number 589 in VP1 of AAV2.

9. A method of producing a gene-transduced cell, the method comprising a step of bringing an AAV particle comprising a mutant of an AAV capsid protein which comprises a peptide comprising an amino acid sequence selected from the group consisting of SEQ ID NOs: 1-7 or a peptide comprising an amino acid sequence that differs from an amino acid sequence selected from the group consisting of SEQ ID NOs: 1-7 by substitution, deletion, insertion and/or addition of one or several amino acids, into contact with a cell.

10. The method according to claim 9, wherein the AAV capsid protein is derived from AAV2.

11. The method according to claim 10, wherein the peptide is placed at a position between amino acid number 588 and amino acid number 589 in VP1 of AAV2.

12. A method of producing an AAV particle, the method comprising using the cell according to claim 5 as a host.

13. A pharmaceutical composition comprising the AAV particle according to any one of claims 6 to 8, and a heterologous polynucleotide harbored inside the AAV particle.
